# EUROPEAN PATENT APPLICATION

(11) **EP 3 272 741 A1**
(43) Date of publication of application: **24.01.2018**
(21) Application number: 16180635.1
(22) Date of filing: 21.07.2016
(51) Int. Cl.: C07D 233/64, A61K 31/625, A61P 1/06, A61P 1/12

(54) **NEW STABLE SOLVATE CRYSTALLINE FORMS OF ELUXADOLINE**

(71) Applicant: Euticals S.P.A., 20122 Milan (IT)
(72) Inventor: PESENTI, Cristina, I-21055 Gorla Minore (VA) (IT); MOLTENI, Marco, I-22032 Albese con Cassano (CO) (IT); LIVIERI, Alessandro, I-27100 Pavia (PV) (IT)
(74) Representative: Pistolesi, Roberto

(57) **Abstract**

Two new stable non stoichiomeric crystalline solvate forms of Eluxadoline with 4-methylpentan-2-one or methyl *tert*-butylether are described as well as the process for the production of the same. The solvate form with 4-methylpentan-2-one is defined as "Eluxadoline form gamma" whereas the solvate with methyl *tert*butylether is defined as "Eluxadoline form delta". Form gamma may be obtained by slurrying Eluxadoline amorphous form in 4-methylpentan-2-one; form delta may be obtained by slurrying Eluxadoline form gamma in methyl *tert*-butylether.

## Description

The present invention relates to two new stable non stoichiomeric crystalline solvate forms of Eluxadoline as well as the process for the production of the same

Eluxadoline (compound identified by the registry number 864821-90-9), whose chemical structure is reported below, is a simultaneous agonist of the µ-opioid receptor and antagonist of the δ-opioid receptor. 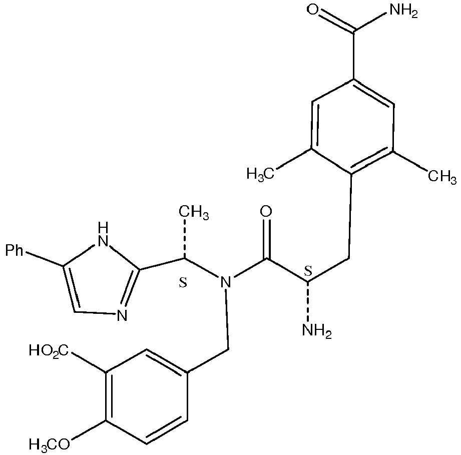 Structural formula of Eluxadoline

The therapeutic use of this molecule was recently approved in the USA for the treatment of diarrhea and abdominal pain in individuals with diarrhea-predominant irritable bowel syndrome without constipating side effects.

This API was disclosed in the international patent application WO2005090315 on 2005 and is formulated in tablets for oral administration. In WO2005090315 Eluxadoline was isolated as dihydrochloride salt, the structure of the product was confirmed by mass spectroscopy but no data were reported on the possible solid state in which the product was isolated.

Literature data describes for Eluxadoline base the existence of two crystalline forms labeled as a and β (WO2009009480, 2008). These two crystalline forms were identified by characteristic DRX spectra. To the best of our knowledge the crystalline form of the product present on the USA market with the trade name of Viberzi^{®} corresponds to Eluxadoline alpha form.

Since it is known that the solid state of an API might influence the bioavailability of a formulation the research of new solid states of an API endowed with better characteristic of stability, consistency and delivery properties has become an important research field for APIs administered in a solid oral form.

### Brief description of the figures

Figure 1 shows reaction scheme 1 for manufacturing the amorphous form of (S)-methyl 2-methoxy-5-(((1-(4-phenyl-1*H*-imidazol-2-yl) ethyl) amino) methyl) benzoate from compound 1
Figure 2 shows reaction scheme 2 for manufacturing the amorphous form of Eluxadoline from (S)-methyl 2-methoxy-5-(((1-(4-phenyl-1*H*-imidazol-2-yl) ethyl) amino) methyl) benzoate
Figure 3 shows reaction scheme 3 for manufacturing the amorphous form of (S)-methyl 2-methoxy-5-(((1-(4-phenyl-1*H*-imidazol-2-yl) ethyl) amino) methyl) benzoate from compound 2
Figure 4 shows the DRX spectrum of Amorphous Eluxadoline
Figure 5 shows the DRX spectrum of Eluxadoline crystalline form gamma measured on a representative sample

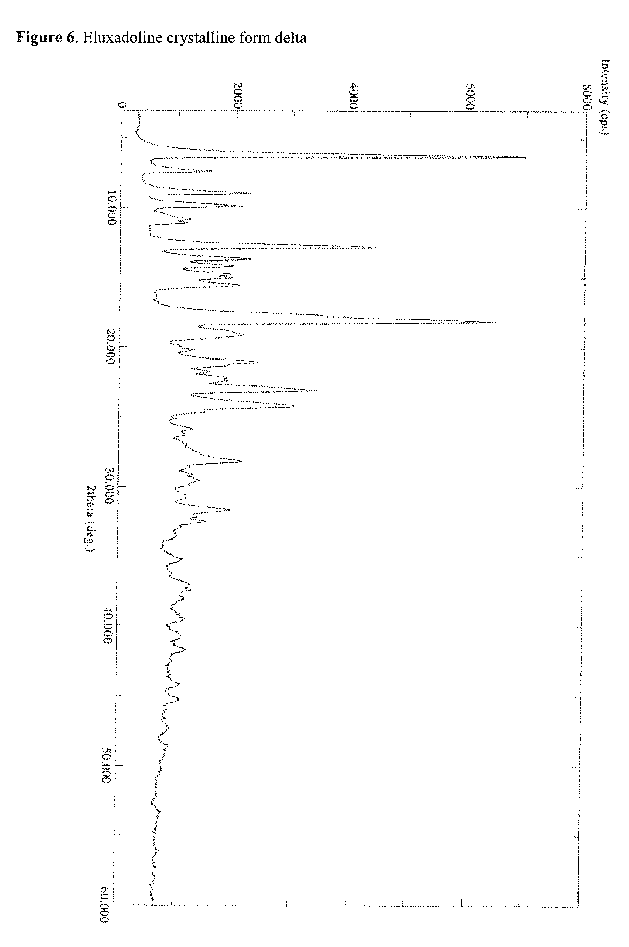

Figure 6 shows the DRX spectrum of Eluxadoline crystal form delta measured on a representative sample
Figure 7 shows the DRX spectrum of Eluxadoline crystal form gamma measured on a representative sample before and after mechanical compression

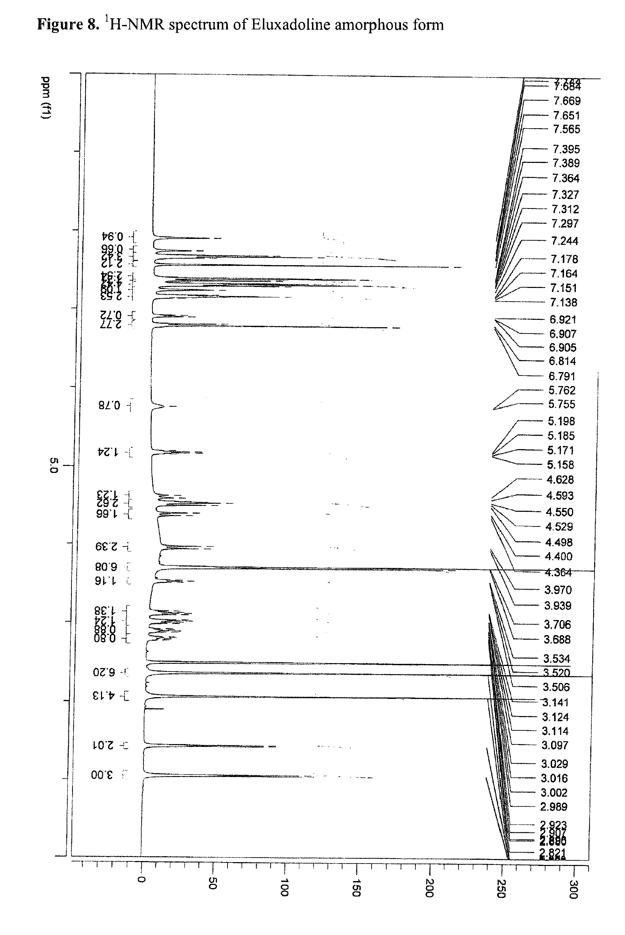

Figure 8 shows the ¹H NMR spectrum of Amorphous Eluxadoline

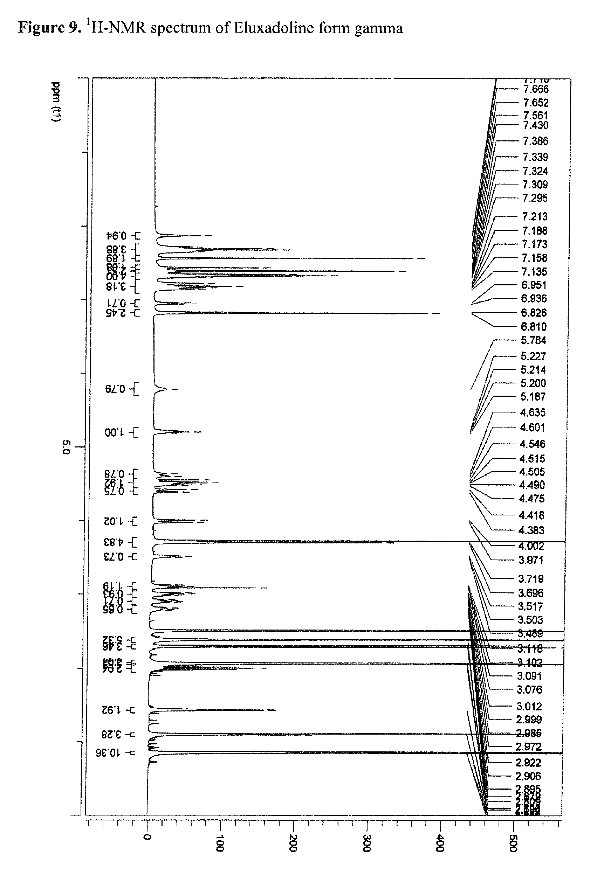

Figure 9 illustrate the ¹H NMR spectrum of Eluxadoline crystalline form gamma registered on a representative sample

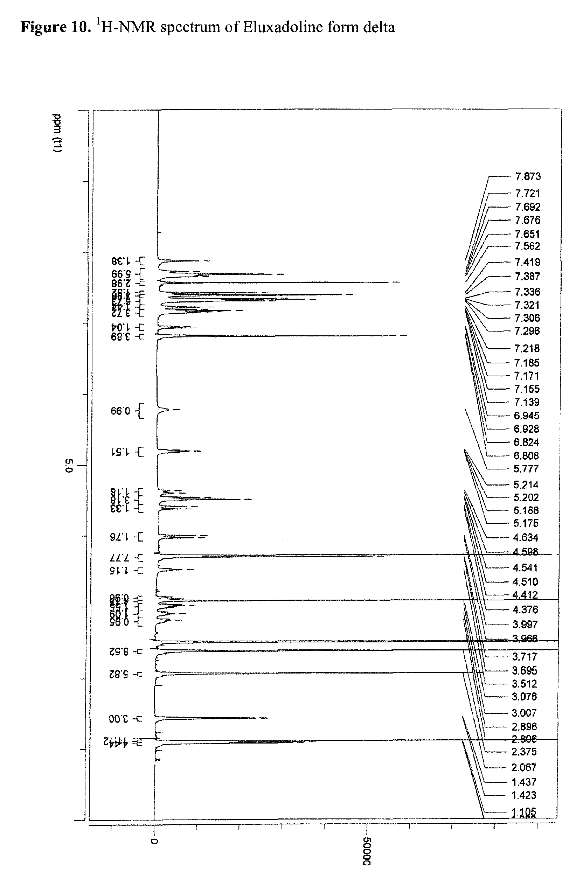

Figure 10 shows the ¹H NMR spectrum of Eluxadoline crystal form delta registered on a representative sample

### Detailed description of the invention

The present invention is directed to new polymorphic forms of Eluxadoline (chemical name: 5-(((S)-2-ammonio-3-(4-carbamoyl-2,6-dimethylphenyl)-N-((S)-1-(4-phenyl-1H-imidazol-2-yl)ethyl)propanamido) methyl)-2-methoxybenzoate) labeled as gamma and delta forms useful for treating irritable bowel syndrome.

These two crystalline forms show the same parameters of crystalline cell (same lattice) but from a chemical point of view are not stoichiometricly solvated with two different solvents: we have named the solvate with 4-methylpentan-2-one "Eluxadoline form gamma" and the solvate with methyl *tert*butylether "Eluxadoline form delta". The labeling utilized by us for these new crystalline forms of Eluxadoline follows the numbering system utilized by Janssen in WO2009009480, which indicates with the Greek letters alpha and beta the first two crystal forms described on Eluxadoline. The quantitation of the solvents on these non stoichiometric solvates was done by NMR spectroscopy. On the basis of drying experiments carried out on crystalline forms gamma and delta we verified that the complete removal of the solvent, for example by drying these two crystal forms under vacuum at 110 °C, compromise the integrity of the lattice giving Amorphous Eluxadoline.

Crystalline form gamma and delta can be defined "clathrates", namely chemical substances consisting of a lattice that traps or contains molecules of a solvent. In these inclusion compounds the guest molecules (4-methylpentan-2-one or methyl *tert*butylether) are in the cavities of the lattice of the host molecule (Eluxadoline). Without the presence of molecules of solvents the clathrate cavities would collapse giving origin to an amorphous product. The structure of a clathrate requires a minimum amount of hosts to fit into and stabilize the cavities (usually one or none in each cavity) without forming any covalent or hydrogen bonds with the solvents, for this reason the number of molecules of guest (solvent) required to stabilize the crystalline structure is not stoichiometric and may be comprise in a defined range.

In a first embodiment, the present invention comprise Eluxadoline form gamma and delta characterized by a DRX spectra having the following powder X-ray diffraction peaks at about (±0.2 2theta): 6.28, 7.30, 8.9, 9.60, 10.26, 10.9, 12.62, 13.60, 14.10, 15.86, 17.96, 18.62, 19.02, 19.32, 20,08, 21.06, 22.64, 22.98, 23.82, 25.52, 26.12, 27.02, 27.56, 27.84, 29.70, 31.34, 31.62, 32.68 degrees 2 theta.

In another aspect of this invention crystalline Eluxadoline form gamma and crystalline Eluxadoline form delta present respectively a content of 4-methylpentan-2-one and methyl *tert*-butylether in the lattice of the crystal comprised between 5 and 20% by weight.

In another embodiment, the present invention comprises a method of preparation of the crystalline Eluxadoline form gamma by slurry of Eluxadoline amorphous form in 4-methylpentan-2-one. According to preferred embodiments of the invention, the relative ratio between 4-methylpentan-2-one and Eluxadoline amorphous form is comprised in a range between 50 and 20 volume(ml)/weight(mg), the temperature of the slurry is comprised between 40-65 °C, the slurrying time is comprised between 16 and 48 hours and the final drying temperature of the product is comprised between 30 and 60 °C at 18 mmHg.

In a further embodiment, the present invention comprises a method of preparation of the crystalline Eluxadoline form delta by slurry of Eluxadoline gamma form in methyl *tert-*butylether. According to preferred embodiments of the invention, the relative ratio between methyl *tert*butylether and Eluxadoline form gamma is comprised in a range between 50 and 30 volume(ml)/weight(mg), the temperature of the slurry is comprised between 40-60 °C, the slurrying time is comprised between 12 and 18 hours and the final drying temperature of the product is comprised between 30 and 60 °C at 18 mmHg.

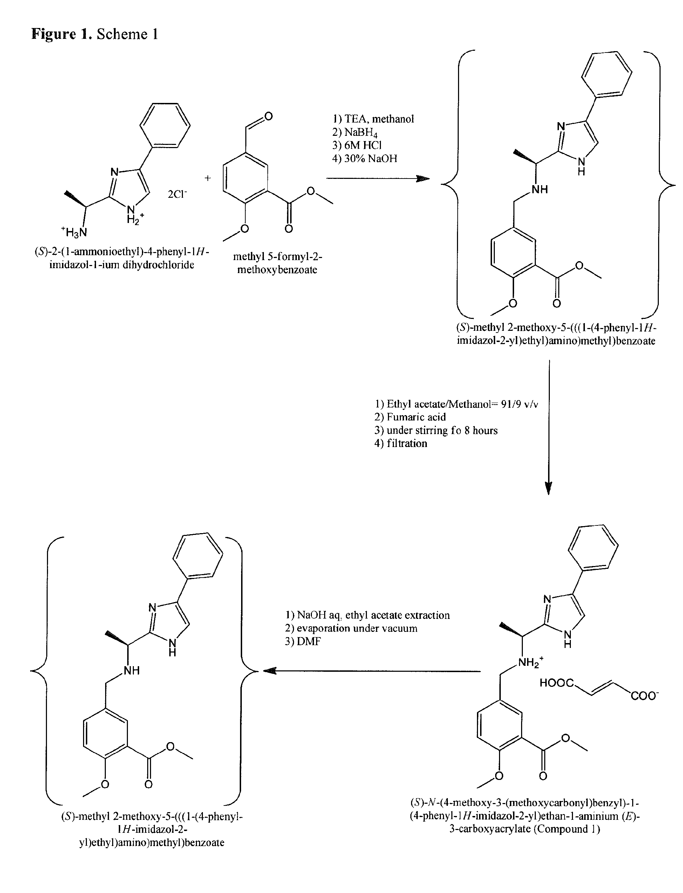

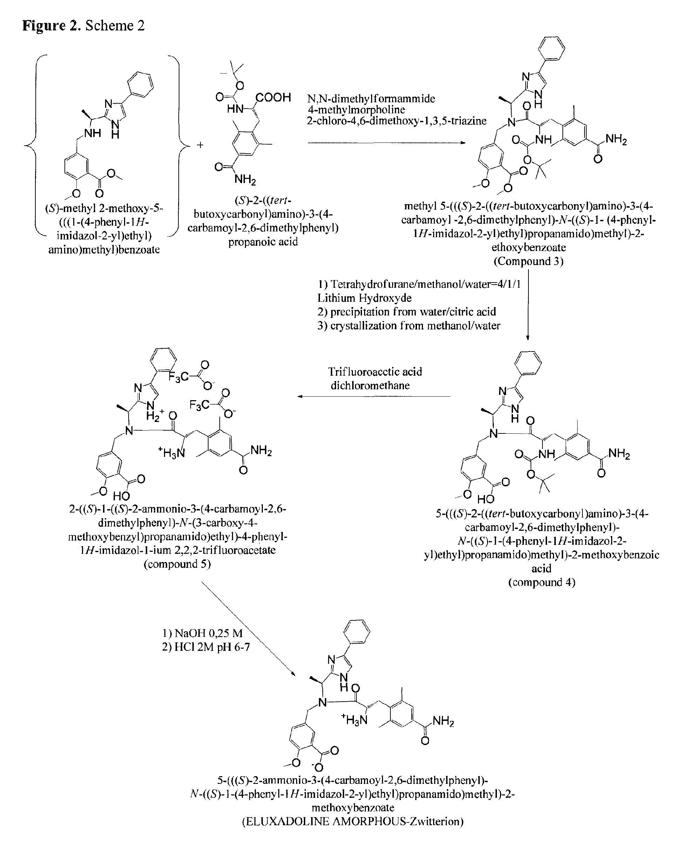

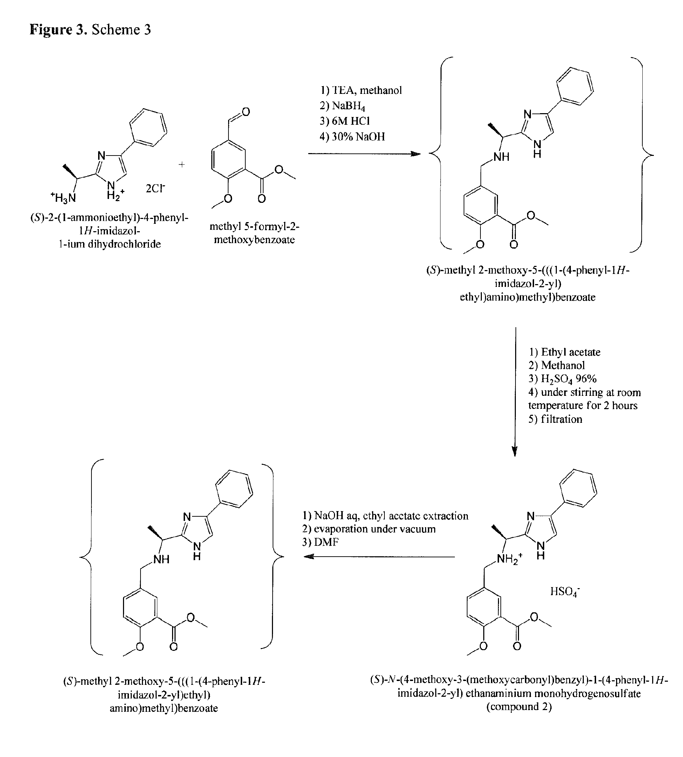

For the purposes of the present invention, Eluxadoline amorphous form which is used for obtaining Eluxadoline form gamma may be prepared according to the process reported in synthetic schemes 1, 2 and 3 (see Figures 1, 2 and 3).

In another embodiment the present invention provides the preparation of Eluxadoline amorphous form following the synthetic schemes 1 and 2 by using as intermediate of the process the new compound 1 or alternatively the corresponding hydrogenosulfate salt (compound identified by chemical name (S)-N-(4-methoxy-3-(methoxycarbonyl)benzyl)-1-(4-phenyl-1H-imidazol-2-yl)ethanaminium hydrogensulfate; Compound 2). The synthetic schemes 1 and 2 follow the process already disclosed in WO2005090315 but with the following relevant improvements:
- the isolation of compounds 1 or 2 (new salts with fumaric acid and hydrogenosulfate of the known compound (S)-methyl 2-methoxy-5-(((1-(4-phenyl-1H-imidazol-2-yl)ethyl)amino)methyl)benzoate); these changes give us the possibility to obtain a more stable and pure compound respect to the corresponding free base;
- the coupling between compound 1 and (S)-2-((tert-butoxycarbonyl)amino)-3-(4-carbamoyl-2,6-dimethylphenyl)propanoic acid is realized in different experimental conditions using the reagent 2-chloro-4,6-dimethoxy-1,3,5-triazine (CDMT) instead of N-(3-Dimethylaminopropyl)-N-ethylcarbodiimide hydrochloride (EDC) with relevant advantages in the yields of this synthetic step. In fact in these experimental conditions we obtained in this step molar yield of about 90% while, according to literature data, an analogous step is realized with EDC with 50% molar yields.

Compound 4 was obtained by basic hydrolysis of compound 3 in an organic aqueous mixture at 15-25 °C. The preferred concentration of compound 3 in the reaction mixture is comprise between 0,1 and 0,3M in a tetrahydrofurane/methanol/water mixture = 4/1/1=v/v/v and the employed base may be selected among sodium hydroxide, lithium hydroxide, potassium hydroxide, potassium carbonate preferably lithium hydroxide used with a five fold molar excess respect to compound 3. After the completion of the reaction (6÷10 hours) the reaction mixture was concentrated under vacuum to about 1/3 of the initial volume and diluted with seven volumes of water. The pH of the reaction was corrected to a final value of 5÷6 with 2M aqueous citric acid solution and the obtained precipitate recovered by suction.

Compound 4 was transformed into the corresponding compound 5 by treatment with a large molar excess of trifluoroacetic acid in dicloromethane solution at room temperature. The preferred molar excess of trifluoroacetic acid respect compound 4 is comprise between 25 and 35 equivalents, preferably 30 equivalents and the concentration of compound 4 in the reaction mixture is comprise between 0,05 and 1M preferably 0,1M.

At the completion the reaction mixture is concentrated to residue and the obtained solid dispersed in Methyl tert-buthyl ether (9÷11 volumes (ml) respect to the initial weight among (g) of compound 4) under stirring for 1÷2 hours to afford a solid recovered by suction.

Amorphous Eluxadoline was obtained from crude compound 5 by dissolving the same in an aqueous basic solution having a pH from 8 to 12, preferably a 0,25M Sodium hydroxide solution, and then acidifying the reaction mixture with 2M Hydrochloric acid till a final pH value comprised between 5÷8. The precipitate so obtained is recovered by filtration, washed on the filter with water, and dried under vacuum at about 30°C for 20 to 28 hours, preferably about 24 hours, to afford amorphous Eluxadoline. The concentration of crude compound 5 in the aqueous basic solution is comprised between 0,03 and 0,1M preferably 0,06M and the final pH value, obtained after addiction of hydrochloric acid 2M, is preferably in the range of 6÷7.

More into details compound 1 free base can be obtained by treatment of the corresponding fumarate (compound 1) or hydrogenosulfate salt (compound 2) by treatment with an excess of aqueous inorganic base (about 10 equivalents of base respect to compound 1), selected by potassium hydroxide and sodium hydroxide, in an organic solvent, selected among ethyl acetate, n-hexane, n-heptane, toluene or a mixture of the same, followed by the separation of the organic solvent, removal of the same by evaporation under vacuum, and dissolution of the obtained residue with N,N-dimethylformamide to a final concentration comprise between 1 and 0,5 M. More preferably the selected inorganic base is sodium hydroxide, the organic solvent is ethyl acetate and the final concentration of compound 1 free base in N,N-dimethylformamide is 0,7 M.

The coupling between compound 1 free base in N,N-dimethylformamide solution and (S)-2-((tert-butoxycarbonyl)amino)-3-(4-carbamoyl-2,6-dimethylphenyl)propanoic acid is carried out by dissolving under stirring (S)-2-((tert-butoxycarbonyl)amino)-3-(4-carbamoyl-2,6-dimethylphenyl)propanoic acid in N,N-dimethylformamide at a concentration comprise between 0,2 and 0,5 M at a temperature comprise between 0 and 10 °C and by adding in sequence 1,3-2,0 equivalent of N-methyl morpholine and 1,3-2,0 equivalent of 2-chloro-4,6-dimethoxy-1,3,5-triazine (CDMT) and maintaining under stirring the reaction for a period of time comprise between 1 and 2 hours. Then, maintaining the reaction under stirring in a range of temperature comprised between -5 and +10 °C, 0,96 equivalent of compound 1 free base in base in N,N-dimethylformamide solution (as above specified) is added in a period of time comprise between 20 and 30'. The reaction mixture was maintained under stirring in a range of temperature comprise between 10 and 25 °C for a period of time comprise between 16-24 hours then water (4-5 volumes respect to the total volume of DMF present in the reaction) was added and the stirring and the temperature of reaction maintained for an additional time of 1-2 hours. The obtained precipitate was recovered by suction affording compound 3 with molar yields comprise between 88-84%. More preferably the concentration of the solution of (S)-2-((tert-butoxycarbonyl)amino)-3-(4-carbamoyl-2,6-dimethylphenyl)propanoic acid in N,N-dimethylformamide is 0,36 M and the obtained solution is cooled down at 0-5 °C, then 1,5 equivalent of N-methyl morpholine and 2-chloro-4,6-dimethoxy-1,3,5-triazine (CDMT) are added and maintained under stirring for 1 hour.

In another embodiment, the present invention relates to the new intermediates which can be used in the preparation of amorphous Eluxadoline, namely (S)-N-(4-methoxy-3-(methoxycarbonyl)benzyl)-1-(4-phenyl-1H-imidazol-2-yl) ethanaminium (E)-3-carboxyacrylate (Compound 1) and (S)-N-(4-methoxy-3-(methoxycarbonyl)benzyl)-1-(4-phenyl-1H-imidazol-2-yl)ethanaminium hydrogensulfate (Compound 2).

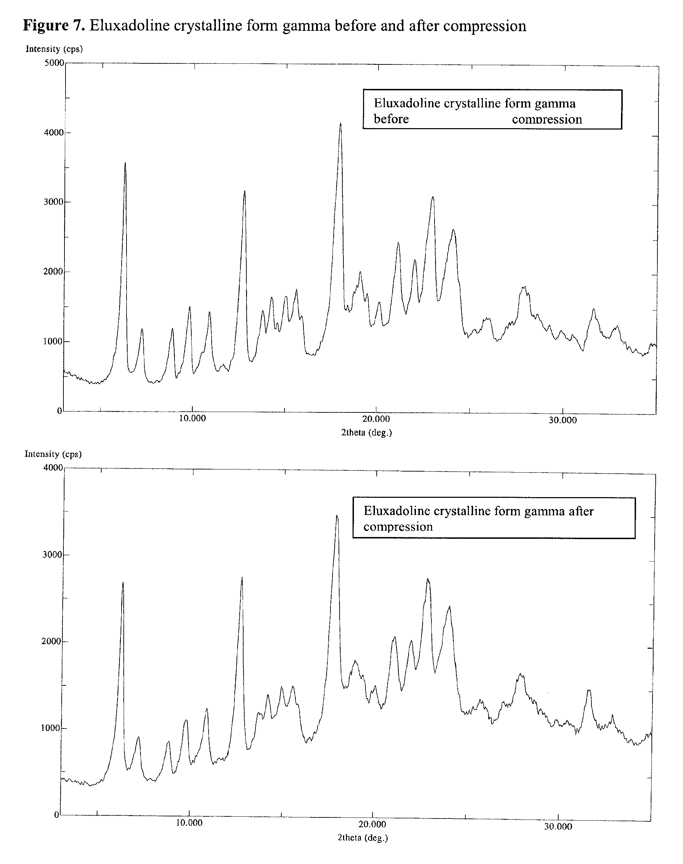

In another embodiment the present invention comprise the use of Eluxadoline form gamma and delta for the preparation of solid formulation. In fact the new crystalline forms of Eluxadoline are stable at room temperature and to a process of compression up to 20 Atm (see Figure 7). This last aspect is relevant since the direct compression is the most common physical treatment carried out on an API in order to prepare compressed tablets.

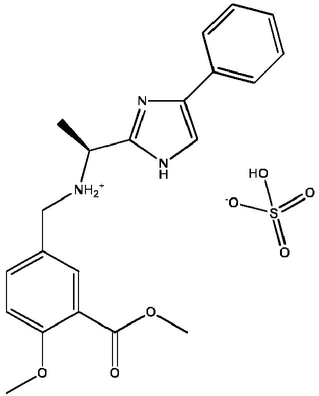

### (S)-N-(4-methoxy-3-(methoxycarbonyl)benzyl)-1-(4-phenyl-1H-imidazol-2-yl)ethanaminium hydrogensulfate (Compound 2)

### Definitions

Unless otherwise defined, all terms of art, notations and other scientific terminology used herein are intended to have the meanings commonly understood by those of skill in the art to which this disclosure pertains. In some cases, terms with commonly understood meanings are defined herein for clarity and/or for ready reference; thus, the inclusion of such definitions herein should not be construed to represent a substantial difference over what is generally understood in the art.

The term "physiologically acceptable excipient" herein refers to a substance devoid of any pharmacological effect of its own and which does not produce adverse reactions when administered to a mammal, preferably a human. Physiologically acceptable excipients are well known in the art and are disclosed, for instance in the Handbook of Pharmaceutical Excipients, sixth edition 2009*,* herein incorporated by reference.

The terms "approximately" and "about" herein refers to the range of the experimental error, which may occur in a measurement.

The terms "comprising", "having", "including" and "containing" are to be construed as openended terms (i.e. meaning "including, but not limited to") and are to be considered as providing support also for terms as "consist essentially of", "consisting essentially of", "consist of" or "consisting of".

The terms "consist essentially of", "consisting essentially of" are to be construed as a semi-closed terms, meaning that no other ingredients which materially affects the basic and novel characteristics of the invention are included (optional excipients may thus included).

The terms "consists of", "consisting of" are to be construed as a closed term.

The following examples disclose the invention without however limiting it.

### Materials and methods

Monodimensional ¹H-NMR spectra have been registered on a Bruker AM 500 instrument (500 MHz) (solvents and temperatures of the analysis are detailed in the experimental section for each example).

The X-rays powder diffractograms (DRX) have been registered on a Rigaku diffractometer (mod. Miniflex) equipped with copper anode (CuKα = 1.54060 Å and CuKα = 1.54439 Å) in the acquisition range [1/2 2θ] of 3.000 to 60.000. With the unique exception of the tablet prepared by compression of crystalline Eluxadoline form gamma (grinded in a mortar before to proceed with the analysis) all the examined samples have been analyzed without any physical treatment.

### Example 1.

### Preparation of (S)-N-(4-methoxy-3-(methoxycarbonyl)benzyl)-1-(4-phenyl-1H-imidazol-2-yl) ethanaminium (E)-3-carboxyacrylate. (Compound 1)

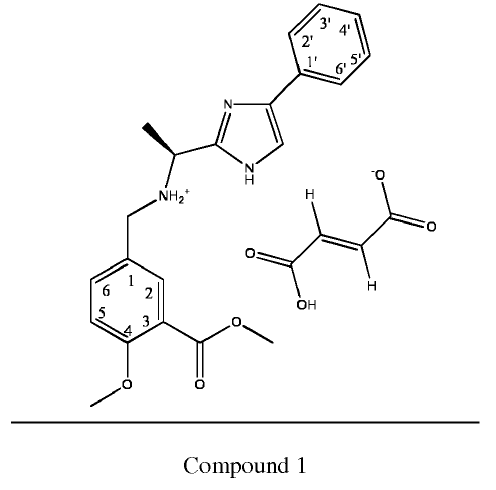

50g (82% of assay; 157,6) of (S)-2-(1-ammonioethyl)-4-phenyl-1H-imidazol-1-ium dihydrochloride were dissolved in methanol (385 ml) under stirring at room temperature, The obtained solution was cooled down to 0-5 °C and triethylamine (3,37 ml; 531,6 mmoles) was added portion wise maintaining the temperature fo the reaction mixture below 10°C. Methyl 5-formyl-2-methoxybenzoate (31 g; 159,6 mmoles) was then added to the reaction mixture by mean of a dropping funnel (at the end of the addiction the funnel was washed with 100 ml of methanol and the washing solution added to the reaction mixture). The reaction temperature was brought to 20-25 °C and maintained under stirring at this temperature for 3,5 hours. After this period the reaction was cooled to 0-4 °C and sodium borohydride (5,4g; 142,59 mmoles) was added portion wise in about 45'. After this period HCl 6M (126 ml) and water (185 ml) were sequentially added to the reaction mixture maintaining the temperature below 10°C. The reaction mixture was maintained under stirring at 15-18 °C for 16 hours and then concentrated by evaporation under vacuum to a final volume of about 330 ml. The aqueous residue so obtained was cooled down to 0-5 °C, the pH corrected with NaOH 30% (about 70 ml) to a final value of 10, extracted with ethyl acetate (2 x 300 ml). The extracted organic phase was concentrated under vacuum to a final volume of about 270 ml (the assay value of this solution is of 52,2 g of (S)-methyl 2-methoxy-5-(((1-(4-phenyl-1H-imidazol-2-yl) ethyl)amino)methyl)benzoate equivalent to about 142,8 mmoles). The obtained solution was added under stirring at room temperature to a suspension of fumaric acid (19,3g; 166 mmoles) in methanol (27 ml) at the temperature of 15-20 °C and then maintained under stirring at 20-25 °C for 18 hours. After this period the obtained suspension was cooled down at 0-5 °C for 1 hour and the precipitate recovered by suction and washed on the filter with ethyl acetate (120 ml) and dried under vacuum at 60 °C to afford 80,73g of compound 1 (assay 82% equivalent to 138 mmoles of product; global molar yields from (S)-2-(1-ammonioethyl)-4-phenyl-1H-imidazol-1-ium dihydrochloride: 87%).
¹HNMR (500 MHz) in deuterated methanol at 298K (δ): 1.71 (3H; d; CH₃-CH), 3.81 (3H; s; Ar-O-CH₃), 3.83 (3H; s; COOCH₃), 3.99-4.12 (2H; dd; Ar-CH₂-NH₂⁺-), 4.46-4.50 (1H; q; CH-CH₃), 6.72 (2H; s; -CH=CH-), 7.11 (1H; d; 5CH aromatic), 7.27 (1H; t; 4'CH aromatic), 7.41 (2H; t; 3' and 5' CH aromatics), 7.46 (1H; s; CH= imidazole), 7.56 (1H; d; 6CH=aromatic), 7.72 (2H; d; 2' and 6' CH aromatics), 7.81 (1H; d; 2CH aromatic).

### Example 2.

### Preparation of (S)-N-(4-methoxy-3-(methoxycarbonyl)benzyl)-1-(4-phenyl-1H-imidazol-2-yl)ethanaminium hydrogensulfate. (Compound 2)

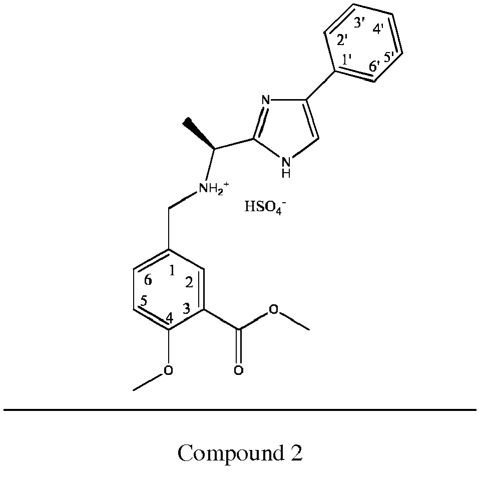

Starting from a solution of (S)-methyl 2-methoxy-5-(((1-(4-phenyl-1H-imidazol-2-yl) ethyl)amino)methyl) benzoate in ethyl acetate, prepared according to the general procedure of example 1 the corresponding hydrogensulfate salt was prepared.

To a solution of (S)-methyl 2-methoxy-5-(((1-(4-phenyl-1H-imidazol-2-yl) ethyl)amino)methyl) benzoate (8,94 g; equivalent to 24,4 mmoles) in ethyl acetate (100ml) under stirring at room temperature were sequentially added methanol (30 ml) and 96% sulfuric acid (2 ml; 37,6 mmoles). The obtained suspension was added drop wise in methyl-*tert* buthyl ether (400 ml) under stirring at room temperature and maintained in these conditions for 2 hours. The obtained precipitate was recovered by suction and washed on the filter with methyl-*tert* buthyl ether (20 ml) and dried under vacuum at 60 °C to afford 11,5g of compound 2 (HPLC purity 98,38%; yields from (S)-methyl 2-methoxy-5-(((1-(4-phenyl-1H-imidazol-2-yl) ethyl)amino)methyl) benzoate: 100%).
¹HNMR (500 MHz) in deuterated methanol at 298K (δ): 1.95 (3H; d; CH₃-CH), 3.75 (3H; s; Ar-O-CH₃), 3.77 (3H; s; COOCH₃), 4.16-4.38 (2H; dd; CH₂-NH₂⁺⁻). 5.03-5.07 (1H; q; CH-CH₃), 7.04 (1H; d; 5CH aromatic), 7.39 (1H; t; 4'CH aromatic), 7.46 (2H; t; 3' and 5' CH aromatics), 7.73 (1H; dd; 6CH=aromatic), 7.76 (1H; s; CH= imidazole), 7.81 (2H; d; 2' and 6' CH aromatics), 7.87 (1H; d; 2CH aromatic).

### Example 3.

### Preparation of methyl 5-(((S)-2-((tert-butoxycarbonyl)amino)-3-(4-carbamoyl-2,6-dimethylphenyl)-N-((S)-1-(4-phenyl-1H-imidazol-2-yl)ethyl)propanamido)methyl)-2-methoxybenzoate. (Compound 3)

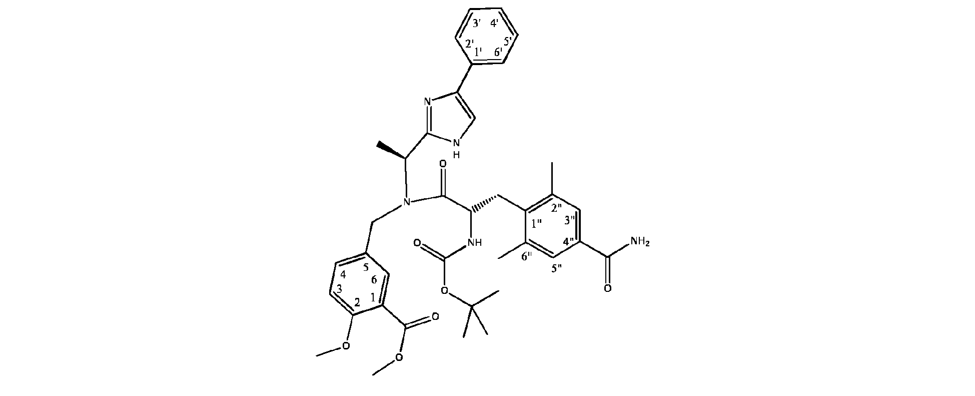

71,63g (148,8 mmoles) of (S)-N-(4-methoxy-3-(methoxycarbonyl)benzyl)-1-(4-phenyl-1H-imidazol-2-yl) ethanaminium (E)-3-carboxyacrylate were suspended under stirring at room temperature in ethyl acetate (300 ml) then water (250 ml) and 30% NaOH (about 200 ml) were sequentially added (final pH value 10). Then the stirring is stopped and the phases were separated; the separated aqueous phase was extracted again with ethyl acetate (310 ml). The collected organic phases were concentrated under vacuum to a final volume of about 100 ml then fresh ethyl acetate (200 ml) was added and the obtained solution evaporated under vacuum to a residue volume of about 65 ml. N, N dimethylformanide (200 ml) was then added and the obtained solution evaporated under vacuum to a final volume of 200 ml (assay value of 50,65g equivalent to 137,64 mmoles of (S)-methyl 2-methoxy-5-(((1-(4-phenyl-1H-imidazol-2-yl)ethyl)amino) methyl) benzoate; 92,52% yields).

48,4g (143,9 mmoles) of (S)-2-((tert-butoxycarbonyl)amino)-3-(4-carbamoyl-2,6-dimethylphenyl)propanoic acid were dissolved under stirring at the temperature of about 4 °C in N,N-dimethylformamide (400 ml) then N-methyl morpholine (23,7 ml; 216 mmoles) was added and the obtained mixture cooled down to 0-5°C. 2-chloro-4,6-dimethoxy-1,3,5-triazine (37,9 g; 216 mmoles) was added potion wise in about 20' and the obtained suspension maintained under stirring at 0-5 °C for about 1 hours. Then, maintain the reaction mixture under stirring at 0-5 °C, the above mentioned solution of (S)-methyl 2-methoxy-5-(((1-(4-phenyl-1H-imidazol-2-yl)ethyl)amino) methyl) benzoate in DMF (assay value of 50,65g equivalent to 137,64 mmoles) was added in about 25'. The reaction mixture was maintained under stirring at 15-20 °C for about 18 hours then was added drop wise in 45-50' in water (2,5 liters) under vigorous stirring at 15-20 °C and at the of the addiction maintained in these conditions for about 1 hour. The obtained suspension was filtered by suction and the precipitate washed on the filter with water (3 x 300 ml) to afford a wet product with an assay value of 73,9 g (108 mmoles) of compound 3 (75% of molar yields from (S)-2-((tert-butoxycarbonyl)amino)-3-(4-carbamoyl-2,6-dimethylphenyl)propanoic acid; HPLC purity 91,4%)
¹HNMR (500 MHz) in deuterated chloroform at 300K:_0.92 (3H; broad s; CH₃-CH), 1.46 (9H; s; (CH₃)₃C-), 2.30 (6H; s; CH₃ aromatics), 2.99-3.03 and 3,32 (2H; 2dd; aromatic-CH₂-CH), 3.61 (3H; 1 broad s; O-CH₃), 3.77 (3H; broad s; COOCH₃), 3.98 and 4.41 (2H; 2 broad signals; Ar-CH₂-N-), 4.79 (1H; broad s; CH-CH₃), 4.99 (1H; broad s; NH-CH-CO), 6.58 (1H; d; 3CH aromatic), 7.08-7,68 (10 H; complex system t; 4CH, 6CH, 2'CH" 3'CGH, 4'CH, 5'CH, 6'CH, 3"CH, 5"CH aromatics and CH= imidazole).

### Example 4.

### Preparation of 5-(((S)-2-((tert-butoxycarbonyl)amino)-3-(4-carbamoyl-2,6-dimethylphenyl)-N-((S)-1-(4-phenyl-1H-imidazol-2-yl)ethyl)propanamido)methyl)-2-methoxybenzoic acid. (Compound 4)

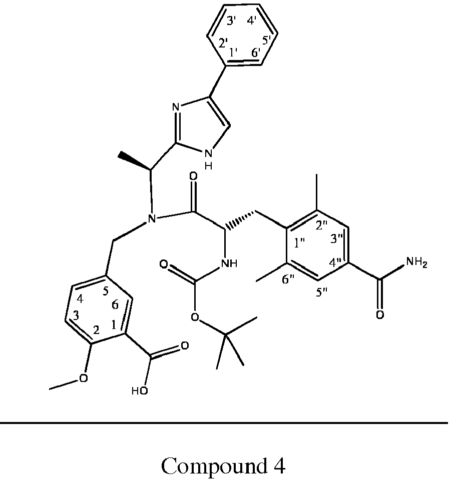

43,5g of methyl 5-(((S)-2-((tert-butoxycarbonyl)amino)-3-(4-carbamoyl-2,6-dimethylphenyl)-N-((S)-1-(4-phenyl-1H-imidazol-2-yl)ethyl)propanamido)methyl)-2-methoxybenzoate (Compound 3; wet product; assay 76,2% corresponding to 48,5 mmoles of dry product) were dissolved under stirring at 18-20°C in a mixture of tetrahydrofurane/methanol/water=4/1/1=v/v/v (242 ml). Lithium hydroxide (6,04g; 251,81 mmoles) was added and the obtained reaction mixture maintained under stirring for about 8 hours at 18-20 °C. Then the reaction mixture was concentrated under vacuum to a residual volume of about 70 ml and the obtained residue diluted under stirring with 500 ml of water, corrected to a final pH value of 5-6 with a 2M aqueous solution of citric acid (about 45 ml) and maintained under stirring at room temperature for 30'. The obtained suspension if filtered on a buckner filter to afford 36,1 g of wet product with an assay value of 85,3% and an HPLC purity of 97,21 (corresponding to 30,71g of compound 4 (45,9 mmoles); molar yields 94,7%).
¹HNMR (500 MHz) in deuterated chloroform at 300K: 0.96 (3H; broad s; CH₃-CH), 1.51 (9H; s; (CH₃)₃C-), 2.14 (6H; s; 2 CH₃ aromatics), 2.98-3.01 and 3.21-3.26 (2H; 2dd; aromatic-CH₂-CH), 3.81 (3H; 1 broad s; O-CH₃), 4.03-4.14 (2H; broad signals; Ar-CH₂-N-), 4.91 (1H; broad s; CH-CH₃), 5.04 (1H; broad s; NH-CH-CO), 6.72 (1H; d; 3CH aromatic), 7.23-7.67 (10 H; complex system t; 4CH, 6CH, 2'CH" 3'CGH, 4'CH, 5'CH, 6'CH, 3"CH, 5"CH aromatics and CH= imidazole).

### Example 5.

### Preparation of 2-((S)-1-((S)-2-ammonio-3-(4-carbamoyl-2,6-dimethylphenyl)-N-(3-carboxy-4-methoxybenzyl)propanamido)ethyl)-4-phenyl-1H-imidazol-1-ium 2,2,2-trifluoroacetate. (Compound 5)

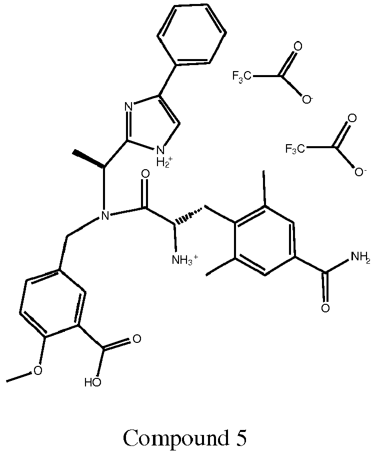

Under inert atmosphere 26,3g of crude compound 4 (assay value 90% corresponding to 23,67g of pure compound 4; 35,43 mmoles) were dissolved under stirring at room temperature in dicloromethane (194 ml). Trifluoroacetic acid (81,1 ml; 1,059 moles) was added portion wise at room temperature according to the following scheme: at time zero (40,6 ml; 530 mmoles), after 2 hours (27 ml; 353 mmoles) and after 17 hours (13,5 ml; 177 mmoles). After the last addiction the reaction mixture was evaporated under vacuum to residue, to the residue dichloromethane was added and evaporated to dryness twice (50 ml each). To the obtained residue methyl *tert*-buthyl ether (237 ml) was added under vigorous stirring to afford a homogeneous dispersion that is maintained under stirring at room temperature for 1 hour. The obtained precipitated was recovered by suction, washed on the filter with was washed with methyl *tert*-buthyl ether (3 x 65 ml), dried under vacuum at 25°C for 12 hours to afford 36,5g of crude compound 5 (HPLC purity=98,9%; 77% assay).

### Example 6.

### Preparation of 5-(((S)-2-ammonio-3-(4-carbamoyl-2,6-dimethylphenyl)-N-((S)-1-(4-phenyl-1H-imidazol-2-yl)ethyl)propanamido)methyl)-2-methoxybenzoate (ELUXADOLINE AMORPHOUS-Zwitterion)

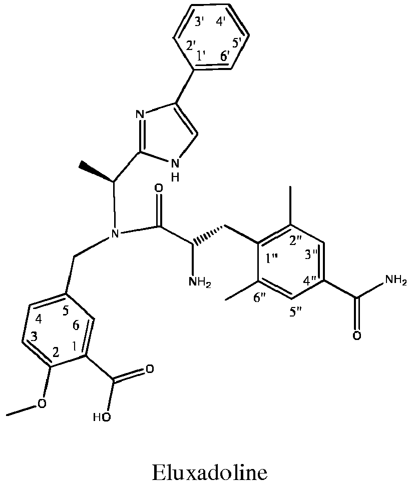

To a 0,25 M solution of sodium hydroxide (550 ml) under stirring at room temperature 36,0 g of crude compound 5 (with and assay value of 27,8 g; 34,9 mmoles) was added portion wise. The obtained turbid solution was stirred at room temperature for 30' then 2M HCl was added in order to reach the pH range 6-7 (about 28 ml were added). The obtained suspension was stirred at room temperature for about 1 hour and then filtered by suction washing the obtained precipitate on the filter with water (2 x 82 ml). The wet solid was then dried under vacuum at 30 °C for 24 hours to give 18,71g of Eluxadoline amorphous-zwitterion (water content 7,62%; HPLC purity of 99,19%; HPLC Assay value on dry substance 96,7%; 84% yields from crude compound 5).

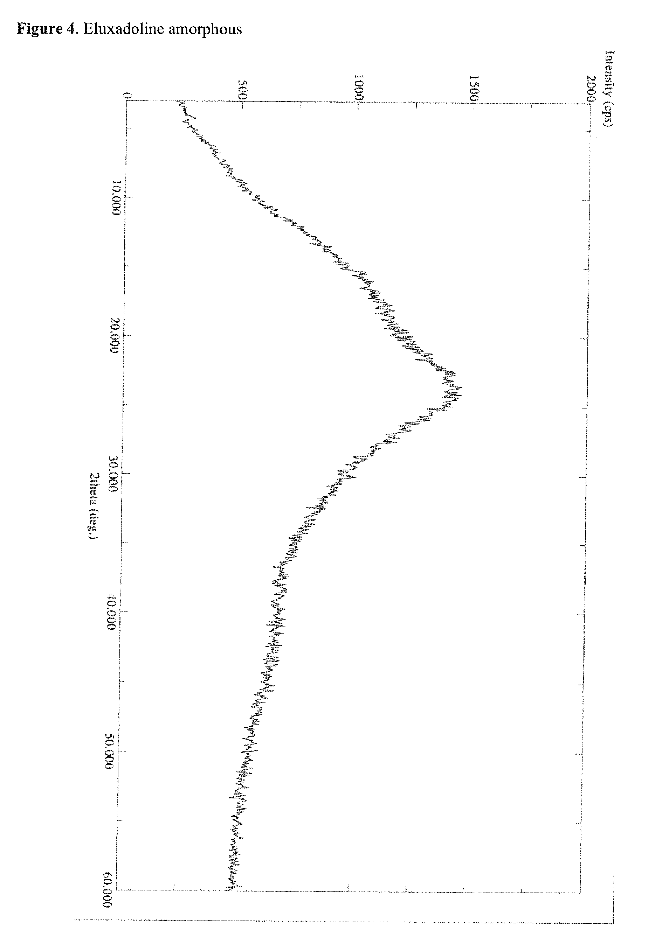

DRX spectroscopy data confirm that the obtained product is an amorphous form (Figure 4).
¹HNMR (500 MHz) in deuterated Dimethylsulfoxide at 298K: 1.05 and 1.43 (3H; 2d; CH₃-CH), 2.07 and 2.37 (6H; 2s; CH₃ aromatics), 2.78-2.92 and 3.00-3.14 (2H; 2 multiplets; aromatic-CH₂-CH), 3.69 and 3.71 (3H; 2s; O-CH₃), 3.51-3.53 and 4.53 (1H; 2 multiplets; ArCH₂-CH-), 3.94-3.97, 4.36-4.40, 4.50-4.55, 4.59-4.63 (2H, multiplet, Ar-CH₂-N), 5.16-5.20 and 5.75 (1H; 2 multiplets; CH-CH₃), 6.79-7.91 (11 H; complex system; 3CH, 4CH, 6CH, 2'CH, 3'CH, 4'CH, 5'CH, 6'CH, 3"CH, 5"CH aromatics and CH= imidazole).

### Example 7.

### Preparation of 5-(((S)-2-ammonio-3-(4-carbamoyl-2,6-dimethylphenyl)-N-((S)-1-(4-phenyl-1H-imidazol-2-yl)ethyl)propanamido)methyl)-2-methoxybenzoate-4-methylpentan-2-one solvate (ELUXADOLINE CRYSTALLINE FORM GAMMA)

500 mg of ELUXADOLINE AMORPHOUS-Zwitterion (0,8 mmoles) were dispersed under stirring at 55 °C in methylisobutylketone (15 ml). After 48 hours the precipitate was recovered by suction, washed on the filter with methylisobutylketone (5 ml) and dried under vacuum at 60 °C to afford 416 mg of Eluxadoline crystalline form gamma. This crystalline compound is a non stoichiometric solvate of Eluxadoline with 4-methylpentan-2-one.
¹HNMR (500 MHz) in deuterated Dimethylsulfoxide at 300K: 0.85 (6H*; d; 2CH₃ of 4-methylpentan-2-one), 1.10 and 1.43 (3H; 2d; CH₃-CH), 2.00-2.06 (4H*; multiplet partially overlapped to a singolet; -CH(CH₃)₂ and CH₃CO of 4-methylpentan-2-one) 2.07 and 2.38 (6H; 2s; CH₃ aromatics), 2.29 (2H*; d; CH₂-CH of 4-methylpentan-2-one), 2.77-2.92 and 2.97-3.12 (2H; 2 multiplets; aromatic-CH₂-CH), 3.70 and 3.72 (3H; 2s; O-CH₃), 3.49-3.52 and 4.49 (1H; 2 multiplets; Ar-CH₂-CH-), 3.97-4.00, 4.38-4.42, 4.51-4.52, 4.60-4.64 (2H, multiplet, Ar-CH₂-N), 5.19-5.23 and 5.78 (1H; 2 multiplets; CH-CH₃), 6.81-7.87 (11 H; complex system; 3CH, 4CH, 6CH, 2'CH, 3'CH, 4'CH, 5'CH, 6'CH, 3"CH, 5"CH aromatics and CH= imidazole). On the basis of the relative integration of the NMR signals of Eluxadoline and 4-methylpentan-2-one on this sample we confirm the presence on the crystal of 15% in weight of 4-methylpentan-2-one.
*note=relative integration of the signals of 4-methylpentan-2-one

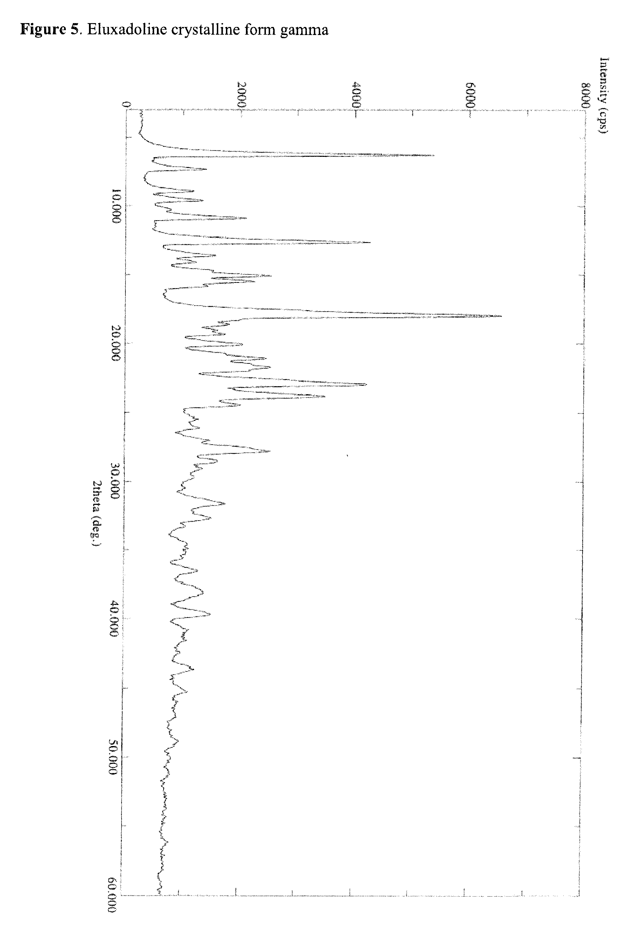

DRX (powder) 2theta values (relative intensity%): 6.28 (83), 7.30 (22), 8.9 (18), 9.60 (21), 10.26 (13), 10.9 (33), 12.62 (66), 13.60 (24), 14.10 (19), 15.86 (22), 17.96 (100), 18.62 (28), 19.02 (25), 19.32 (27), 20,08 (32), 21.06 (38), 22.64 (45), 22.98 (65), 23.82 (54), 25.52 (20), 26.12 (20), 27.02 (23), 27.56 (34), 27.84 (39), 29.70 (19), 31.34 (23), 31.62 (27), 32.68 (24). Figure 5.

DSC registered at an heating rate of 10°C/min from 40 to 250 °C: two endothermic peaks at 175 °C and 186 °C (43,78 J/g).

Thermo gravimetric assay (TGA) registered at an heating rate of 10 °C/min from 40 to 250 °C : 16% of weigh (theoretical equimolar amount: 14,9%).

### Example 8.

### Preparation of 5-(((S)-2-ammonio-3-(4-carbamoyl-2,6-dimethylphenyl)-N-((S)-1-(4-phenyl-1H-imidazol-2-yl)ethyl)propanamido)methyl)-2-methoxybenzoate solvate with methyl tert butylether (ELUXADOLINE CRYSTALLINE FORM DELTA)

2 g of Eluxadoline crystalline form gamma prepared according to the Example 7 were dispersed in methyl *tert* butylether (80 ml) under stirring at the temperature of 50 °C. After 12 hours the suspended solid was recovered by suction, washed on the filter with methyl *tert* butylether (20 ml) and dried under vacuum at 60 °C to afford 1,8 g of Eluxadoline crystalline form delta. This crystalline compound is a non stoichiometric solvate of Eluxadoline with methyl *tert* butylether.
¹HNMR (500 MHz) in deuterated Dimethylsulfoxide at 300K: 1.09 and 1.43 (3H; 2d; CH₃-CH), 1.11 (9H*; s; (CH₃)₃C of methyl *tert*butylether), 2.07 and 2.38 (6H; 2s; CH₃ aromatics), 2.81-2.90 and 3.01-3.08 (2H; 2 multiplets; aromatic-CH₂-CH), 3.08 (3H*; s partially overlapped to the previous system; CH₃O- of methyl *tert*butylether), 3.70 and 3.72 (3H; 2s; O-CH₃), 3.51 and 4.51 (1H; 2 multiplets; Ar-CH₂-CH-), 3.97-4.00, 4.38-4.41, 4.51-4.54, 4.60-4.63 (2H, multiplet, Ar-CH₂-N), 5.19-5.23 and 5.78 (1H; 2 multiplets; CH-CH₃), 6.81-7.87 (11 H; complex system; 3CH, 4CH, 6CH, 2'CH, 3'CH, 4'CH, 5'CH, 6'CH, 3"CH, 5"CH aromatics and CH= imidazole). On the basis of the relative integration of the NMR signals of Eluxadoline and of methyl *tert*butylether on this sample we confirm the presence on the crystal of 7,2% in weight of methyl *tert*butylether.
*note: relative integration of the signals of methyl *tert*butylether

DRX (powder) 2theta values (relative intensity%): 6.34 (100), 7.34 (18), 8.96 (24), 9.42 (26), 10.46 (25), 10.86 (29), 12.78 (48), 13.66 (64), 14.18 (47), 15.70 (51), 17.80 (88), 18.82 (53), 19.04 (55), 19.42 (46), 19,90 (38), 21.06 (59), 22.44 (61), 23.00 (75), 24.00 (60), 25.58 (34), 25.96 (33), 26.82 (35), 27.36 (39), 28,00 (48), 29.52 (36), 31.58 (42), 32.56 (38). Figure 5.

### Example 9.

### Stability data of Eluxadoline crystalline form gamma to the compression.

Eluxadoline crystalline form gamma prepared according to the Example 7 was pressed into a disc under at a pressure of about 20 Atm to afford a tablet. The obtained tablet was examined by DRX (powder) spectroscopy confirming that the solid state is unchanged: the DRX spectrum is practically superimposeable with those registered on the powder before compression (Figure 7).

## Claims

1. Solvate crystalline form of eluxadoline having a DRX spectrum with peaks at about (±0.2): 6.28, 7.30, 8.9, 9.60, 10.26, 10.9, 12.62, 13.60, 14.10, 15.86, 17.96, 18.62, 19.02, 19.32, 20,08, 21.06, 22.64, 22.98, 23.82, 25.52, 26.12, 27.02, 27.56, 27.84, 29.70, 31.34, 31.62, 32.68 degrees 2 theta.

2. Solvate crystalline form of eluxadoline according to claim 1 having a DRX spectrum as shown in figure 5.

3. Solvate crystalline form of eluxadoline according to claim 1 having a DRX spectrum as shown in figure 6.

4. Solvate crystalline form of eluxadoline according to claim 1, which is a 4-methylpentan-2-one solvate.

5. Solvate crystalline form of eluxadoline according to claim 4, wherein the content of 4-methylpentan-2-one in the lattice of the crystal is comprised between 5 and 20% by weight.

6. Solvate crystalline form of eluxadoline according to claim 4, having a ¹H NMR spectrum as shown in figure 9.

7. Solvate crystalline form of eluxadoline according to claim 4, having a DSC spectrum with two endothermic peaks at about 175 °C and about 186 °C.

8. Solvate crystalline form of eluxadoline according to claim 1, which is a methyl *tert*-butylether solvate.

9. Solvate crystalline form of eluxadoline according to claim 8, wherein the content of 4- methyl *tert*-butylether in the lattice of the crystal is comprised between 5 and 20% by weight.

10. Solvate crystalline form of eluxadoline according to claim 8, having a ¹H NMR spectrum as shown in figure 10.

11. A process for the manufacture of a solvate crystalline form of eluxadoline according to anyone of claims 4 to 7, which comprises slurrying Eluxadoline in amorphous form in 4-methylpentan-2-one.

12. A process according to claim 11, wherein the ratio between 4-methylpentan-2-one and Eluxadoline amorphous form is comprised in a range between 50 and 20 volume (ml)/weight(mg).

13. A process according to claim 11, wherein the temperature of the slurry is comprised between 40 and 65 °C.

14. A process for the manufacture of solvate crystalline form of Eluxadoline according to anyone of claims 8 to 10, which comprises slurrying Eluxadoline according to claim 4 in methyl *tert-*butylether.

15. A process according to claim 14, wherein the relative ratio between methyl *tert*butylether and Eluxadoline according to claim 4 is comprised in a range between 50 and 30 volume(ml)/weight(mg).

16. A process according to claim 14, wherein the temperature of the slurry is comprised between 40 and 60 °C.

17. A pharmaceutical composition containing solvate crystalline form of Eluxadoline according to anyone of claims 1 to 10 and at least one physiologically acceptable excipient.

18. A pharmaceutical composition according to claim 17, which is a tablet.

19. Solvate crystalline form of Eluxadoline according to anyone of claims 1 to 10, for use in the treatment of irritable bowel syndrome.

20. A process for the manufacture of Eluxadoline in amorphous form which comprises dissolving a compound of formula 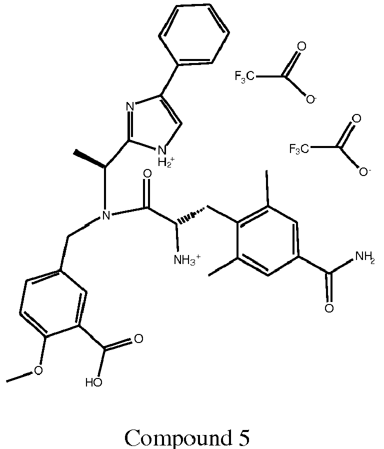 in an aqueous basic solution having a pH from 8 to 12, preferably a sodium hydroxide solution, and acidifying the mixture till a pH value from 5 to 8, preferably from 6 to 7.

21. A process according to claim 20, wherein the concentration in the aqueous basic solution is from 0,03 to 0,1M, preferably about 0,06M.

22. A process according to claim 20, wherein the so obtained precipitate is recovered by filtration, washed with water and dried under vacuum.

23. A compound of formula:

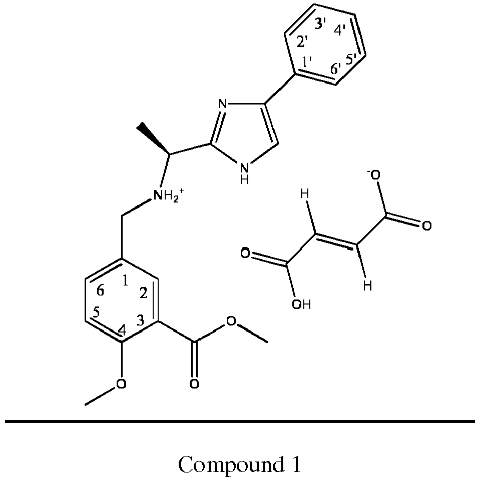

24. A compound of formula:
